# EUROPÄISCHE PATENTANMELDUNG

(11) **EP 2 380 554 A2**
(43) Veröffentlichungstag der Anmeldung: **26.10.2011**
(21) Anmeldenummer: 10195187.9
(22) Anmeldetag: 15.12.2010
(51) Int. Cl.: A61K 8/34, A61Q 5/04, A61Q 5/06, A61Q 5/08, A61Q 5/10, A61Q 5/00

(54) **Haar- und Kopfhautschonende Haafärbe- und Dauerwellmittel**

(30) Priorität: 20.04.2010 DE 102010027949
(71) Anmelder: Henkel AG & Co. KGaA, 40589 Düsseldorf (DE)
(72) Erfinder: Kleen, Astrid, 20457, Hamburg (DE); Hagenow, Susanne, 20359, Hamburg (DE)

(57) **Zusammenfassung**

Gegenstand der Erfindung ist ein Mittel zur Farb- und/oder Formveränderung von keratinischen Fasern, insbesondere menschlichen Haaren, enthaltend in einem kosmetischen Träger mindestens eine farb- und/oder formverändernde Komponente und zusätzlich *trans*-4-*tert*-Butyl-Cyclohexanol. Die erfindungsgemäßen Mittel weisen eine Verbesserung der Hautverträglichkeit und zu einer Verringerung an Irritationen auf der Kopfhaut auf. Gleichzeitig führt die Verwendung von *trans*-4-*tert-*Butyl-Cyclohexanol in Mittel zur Farb- und/oder Formveränderung von menschlichen Haaren zur Verbesserung des Feuchtigkeitshaushaltes, insbesondere der Feuchthaltung keratinhaltiger Fasern, zur Verbesserung der Avivage keratinhaltiger Fasern, insbesondere menschlicher Haare, zur Verbesserung des Glanzes keratinhaltiger Fasern, insbesondere menschlicher Haare, sowie zum Schutz der Destrukturierung keratinhaltiger Fasern, insbesondere menschlicher Haare, durch UV-Strahlung.

## Beschreibung

Gegenstand der Erfindung ist ein Mittel zur Farb- und/oder dauerhaften Formveränderung von keratinischen Fasern, das es in einem kosmetischen Träger mindestens eine farb- und/oder formverändernde Komponente sowie *trans*-4-*tert*-Butyl-Cyclohexanol enthält. Die erfindungsgemäßen Mittel eignen sich insbesondere zur Reduktion der Haarschädigung und zur Verbesserung der Hautverträglichkeit. Weiterhin betrifft die Erfindung ein Verfahren zur Anwendung solcher Mittel auf keratinhaltigen Fasern. Darüber hinaus betrifft die Erfindung die Verwendung des Mittels zur Färbung und/oder dauerhaften Formveränderung mit verringertem Irritationspotential.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Diese Mittel sollen neben der gewünschten Färbe und Formleistung möglichst minimale Schädigungen auf dem Haar hervorrufen und insbesondere eine gute Hautverträglichkeit aufweisen.

Die dauerhafte Verformung von Keratinfasern wird üblicherweise so durchgeführt, dass man die Faser mechanisch verformt und die Verformung durch geeignete Hilfsmittel festlegt. Vor und/oder nach dieser Verformung behandelt man die Faser mit der wässrigen Zubereitung einer keratinreduzierenden Substanz, wobei diese einen Teil der Disulfid-Bindungen des Keratins zu ThiolGruppen, so dass es zu einer Lockerung der Peptidvernetzung spaltet und es infolge der Spannung der Faser durch die mechanische Verformung zu einer Neuorientierung des Keratingefüges kommt, und spült nach einer Einwirkungszeit mit Wasser oder einer wässrigen Lösung. In einem zweiten Schritt behandelt man dann die Faser mit der wässrigen Zubereitung eines Oxidationsmittels, unter dessen Einfluss erneut Disulfid-Bindungen geknüpft werden, und so das Keratingefüge in der vorgegebenen Verformung neu fixiert wird. Nach einer Einwirkungszeit wird auch dieses ausgespült und die Faser von den mechanischen Verformungshilfsmitteln (Wickler, Papilloten) befreit. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten Haaren angewendet werden.

Die permanente, oxidative Färbung sowie die Haarbleiche und die Fixierung im Rahmen der Dauerwelle finden meist als oxidative Haarbehandlung in Gegenwart von Oxidationsmitteln, wie Wasserstoffperoxid, statt. Das Oxidationsmittel erzielt hierbei leider nicht nur die erwünschte kosmetische Wirkung, sondern wirkt sich negativ auf die Stabilität, die Haptik und die Optik der keratinhaltigen Faser, insbesondere der menschlichen Haare, aus. Derart geschädigtes Haar wirkt stumpf und spröde. In extremen Fällen kommt es sogar zum Haarbruch. Bei einer Anwendung solcher oxidativen Haarbehandlungsmittel hegt der bequeme Verbraucher den Wunsch, durch Verwendung eines 2-in-1-Produkts um Folgeanwendungen zur Kaschierung der oben genannten unerwünschten Auswirkungen herumzukommen.

Es besteht das Bedürfnis, Haarpflegeprodukte- insbesondere Haarfärbemittel - weiter zu verbessern und ihnen weitere vorteilhafte Eigenschaften zu verleihen. Dabei sollte insbesondere dem Feuchtigkeitsverlust keratinischer Fasern und der damit einhergehenden Verschlechterung der mechanischen Eigenschaften und der Versprödung Einhalt geboten werden.

Es ist daher eine Aufgabe der vorliegenden Erfindung, die zuvor beschriebenen Nebenwirkungen oxidativer Haarbehandlungen im Rahmen eines 2-in-1-Produktes bereits während der oxidativen Haarbehandlung zu verringern, ohne den Wirkungsgrad des oxidativen Kosmetikums, insbesondere bezüglich Farbintensität, Farbechtheit, Aufhellleistung bzw. Wellwirkung, zu verschlechtern.

Aufgabe der vorliegenden Erfindung ist daher die Bereitstellung kosmetischer Mittel zur Farb- und/oder Formveränderung keratinischer Fasern, die eine verbesserte Haar- und Hautverträglichkeit aufweisen. Es ist insbesondere Aufgabe der vorliegenden Anmeldung, das Irritationspotential für die Haut zu senken und so eine Minimierung des Allergiepotentials zu erzielen. Weiterhin ist es Aufgabe der vorliegenden Erfindung, ein Mittel bereitzustellen, welches den Einsatz von erhöhten Mengen an Oxidationsfarbstoffvorprodukten und/oder Oxidationsmittel erlauben, so daß die Einwirkzeit dieser Mittel verkürzt werden kann, ohne daß die erhöhte Konzentration dieser Reizstoffe zu Irritationen und Reizungen der Kopfhaut führt oder zu einer übrmäßigen Schädigung der keratinischen Fasern führt.

Es konnte nun gefunden werden, daß farb- und/oder formverändernde Mittel, die neben einer farb- und/oder formverändernden Komponente *trans*-4-*tert*-Butyl-Cyclohexanol enthalten, die oben genannten Nachteile vermeiden. Bedingt durch die Schutzwirkung bei Anwendung des erfindungsgemäßen Mittels kann die Haar- und Hautverträglichkeit hierdurch deutlich verbessert werden. Zusammensetzungen, die *trans*-4-*tert*-Butyl-Cyclohexanol enthalten, sind beispielsweise aus der WO2009/087242 A2 bekannt. Diese Schrift betrifft aber ausschließlich Zusammensetzungen für die Dermalanwendung, es findet sich allerdings darin kein Hinweis auf kosmetische Mittel zur Farb- und/oder Formveränderung.

Es hat sich gezeigt, daß *trans*-4-*tert*-Butyl-Cyclohexanol dazu geeignet ist, in der Haut, insbesondere der Kopfhaut, die nucleare Translocation und Aktivierung von des Transkriptionsfaktors NF-κB (nuclearer Faktor κB / (kappa)B) sowie die Produktion von entzündungsverstärkenden Prostaglandinen, insbesondere PGE2, in den Keratinocyten zu reduzieren. Darüber hinaus begünstigt *trans*-4-*tert*-Butyl-Cyclohexanol die Bildung des Hormons α-MSH (alpha-Melanocyten-stimulierendes Hormon), welches eine entscheidende Rolle bei der Regulation und Verringerung von entzündlichen Empfindungen, insbesondere des Juckreizes, spielt. Dadurch wird insgesamt die Toleranzschwelle der Haut, auf irritierende Substanzen mit Reizung und Entzündungen zu reagieren, herabgesenkt. Somit werden sowohl Entzündungspotential wie auch Schmerzempfindung der Kopfhaut reduziert. Außerdem kann sich angegriffene, empfindliche Haut beruhigen und so potentiell allergienauslösende Substanzen besser tolerieren. Insgesamt führt die Verwendung von *trans*-4-*tert*-Butyl-Cyclohexanol in Mittel zur Farb- und/oder Formveränderung von menschlichen Haaren zu einer Verbesserung der Hautverträglichkeit und zu einer Verringerung an Irritationen auf der Kopfhaut. Gleichzeitig führt die Verwendung von *trans*-4-*tert*-Butyl-Cyclohexanol in Mittel zur Farb- und/oder Formveränderung von menschlichen Haaren zur Verbesserung des Feuchtigkeitshaushaltes, insbesondere der Feuchthaltung keratinhaltiger Fasern, zur Verbesserung der Avivage keratinhaltiger Fasern, insbesondere menschlicher Haare, zur Verbesserung des Glanzes keratinhaltiger Fasern, insbesondere menschlicher Haare, sowie zum Schutz der Destrukturierung keratinhaltiger Fasern, insbesondere menschlicher Haare, durch UV-Strahlung.

Ein erster Gegenstand der Erfindung ist daher ein Mittel zur Farb- und/oder Formveränderung von keratinischen Fasern, insbesondere menschlichen Haaren, enthaltend in einem kosmetischen Träger mindestens eine farb- und/oder formverändernde Komponente und zusätzlich *trans*-4-*tert-*Butyl-Cyclohexanol.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist im Sinne der Erfindung ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Erfindungsgemäße Mittel können als Mittel zum Färben und/oder als Mittel zum Aufhellen keratinischer Fasern oder als Mittel zur Formveränderung keratinischer Fasern konfektioniert werden. Mittel zum Aufhellen keratinischer Fasern enthalten üblicherweise mindestens ein Oxidationsmittel, Mittel zum Färben enthalten mindestens einen direktziehende Farbstoff und/oder Oxidationsfarbstoffvorprodukte. Mittel, die gleichzeitig färbend und aufhellend wirken, enthalten meist sowohl Oxidationsmittel als auch mindestens einen direktziehende Farbstoff und/oder Oxidationsfarbstoffvorprodukte. Sind nur direktziehende Farbstoffe vorhanden, ist beispielsweise auch die Formulierung eines Färbeshampoos möglich.

Bevorzugte erfindungsgemäße Mittel sind Färbemittel, d.h. Mittel zur Veränderung der Farbe keratinischer Fasern. Unter diesen sind insbesondere die so genannten Oxidationsfärbemittel bevorzugt. Die erfindungsgemäßen Oxidationsfärbemittel enthalten mindestens eine Kuppler- und mindestens eine Entwicklerkomponente. Kuppler- und Entwicklerkomponenten werden auch als Oxidationsfarbstoffvorprodukte bezeichnet. Zudem können die erfindungsgemäßen Oxidationsfärbemittel auch noch direktziehende Farbstoffe als Nuanceure enthalten. Erfindungsgemäß bevorzugte Mittel zum Färben und/oder Aufhellen keratinischer Fasern sind demnach dadurch gekennzeichnet, daß sie zusätzlich - bezogen auf ihr Gewicht - 0,001 bis 5 Gew.-% eines oder mehrerer Oxidationsfarbstoffvorprodukte und/oder direktziehender Farbstoffe enthalten. Diese werden weiter unten detailliert beschrieben.

Die erfindungsgemäßen Mittel enthalten als Pflegesubstanz zur Verhinderung oder zum Ausgleich von Feuchtigkeitsverlust und zur Verbesserung der mechanischen Eigenschaften, insbesondere zur Verhinderung oder zum Ausgleich von Sprödigkeit der keratinischen fasern sowie zur Kopfhautschonung *trans*-4-*tert*-Butyl-Cyclohexanol.

Im Rahmen der vorliegenden Erfindung umfaßt die Bezeichnung *trans*-4-*tert*-Butyl-Cyclohexanol sowohl das (+)-*trans*-4-*tert*-Butyl-Cyclohexanol der Formel (I), als auch das (-)-*trans*-4-*tert*-Butyl-Cyclohexanol der Formel (II) als auch deren Mischungen, insbesondere deren racemisches Gemisch.

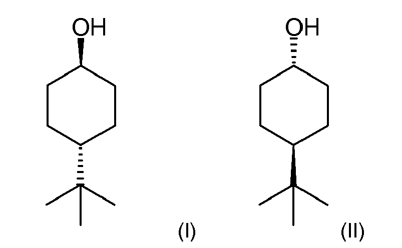

In besonders bevrozugten Mitteln wird *trans*-4-*tert*-Butyl-Cyclohexanol in Mengen oberhalb von 0,01 Gew.-% eingesetzt. Bevorzugte erfindungsgemäße Mittel sind dadurch gekennzeichnet, daß sie - bezogen auf ihr Gewicht - 0,05 bis 10 Gew.-%, vorzugsweise 0,1 bis 7,5 Gew.-%, weiter bevorzugt 0,25 bis 5 Gew.-%, besonders bevorzugt 0,5 bis 2,5 und insbesondere 0,5 bis 1,25 Gew.-% *trans*-4-*tert*-Butyl-Cyclohexanol enthalten.

Die erfindungsgemäßen Mittel werden besonders vorteilhaft zur Farbveränderung keratinischer Fasern eingesetzt. Erfindungsgemäß bevorzugte Mittel sind daher dadurch gekennzeichnet, das Mittel als farbverändernde Komponente mindestens ein Oxidationsfarbstoffvorprodukt und/oder mindestens einen direktziehenden Farbstoff und/oder mindestens einer Vorstufe naturanaloger Farbstoffe und/oder mindestens ein Aufhellmittel enthalten.

In einer ersten Ausführungsform werden die farbverändernden Komponenten ausgewählt aus Oxidationsfarbstoffvorprodukten.

Die Oxidationsfarbstoffvorprodukte werden bevorzugt in einer Menge von 0,005 bis 25 Gew.-%, bevorzugt von 0,05 bis 20 Gew.-% und besonders bevorzugt von 0,1 bis 15 Gew.-%, jeweils bezogen auf das anwendungsbereite Oxidationsfärbemittel, verwendet.

Ganz besonders bevorzugte erfindungsgemäße Mittel sind dadurch gekennzeichnet, daß sie als farbverändernde Komponente mindestens ein Oxidationsfarbstoffvorprodukt enthalten, wobei der Gewichtsanteil aller Oxidationsfarbstoffvorprodukte zwischen 0,001 und 20 Gew.-%, bevorzugt zwischen 0,1 und 10 Gew.-% und insbesondere zwischen 0,2 und 5,0 Gew.-%, jeweils bezogen auf das Gesamtgewicht des anwendungsbereiten Mittels, liegt.

In einer ersten bevorzugten Ausführungsform enthält das Färbemittel mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Bevorzugte Entwicklerkomponenten werden im Prioritätsdokument detailliert beschrieben. Erfindungsgemäß bevorzugte Mittel zum Färben und/oder Aufhellen keratinischer Fasern sind dadurch gekennzeichnet, daß sie als Oxidationsfarbstoffvorprodukt mindestens eine Entwicklerkomponente enthalten, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol, Hydroxyethyl-p-Phenylendiamin, 1,10-Bis-(2',5'-diaminophenyl)-1,4,7,10-tetraoxadecan, 4-Aminophenol, 4-Amino-3-methylphenol, Bis-(5-amino-2-hydroxyphenyl)methan, 2,4,5,6- Tetraaminopyrimidin, 2-Hydroxy-4,5,6- triaminopyrimidin, 4,5-Diamino-1-(2-hydroxyethyl)-pyrazol.

Die erfindungsgemäßen Färbemittel können weiterhin mindestens eine Kupplerkomponente enthalten. Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Bevorzugte Entwicklerkomponenten werden im Prioritätsdokument detailliert beschrieben. Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-((3-[(2-Hydroxyethyl)amino]-4-methoxy-5-methylphenyl)amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Erfindungsgemäß bevorzugte Mittel zum Färben und/oder Aufhellen keratinischer Fasern sind dadurch gekennzeichnet, daß sie als Oxidationsfarbstoffvorprodukt mindestens eine Kupplerkomponente enthalten, wobi bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

Vorzugsweise werden Kuppler- und Entwicklerkomponenten in einem bestimmten Verhältnis zueinander eingesetzt. Hier sind erfindungsgemäße Oxidationsfärbemittel bevorzugt, die die Kupplerkomponente(n) in einer Menge von 0,01 bis 20 Gew.-%, vorzugsweise von 0,5 bis 5 Gew.-%, und die Entwicklerkomponente(n) in einer Menge von 0,01 bis 20 Gew.-%, vorzugsweise von 0,5 bis 5 Gew.-%, jeweils bezogen auf das gesamte Oxidationsfärbemittel, enthalten.

Zusätzlich können die Färbemittel zur Nuancierung einen oder mehrere direktziehende Farbstoffe enthalten.

Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

Erfindungsgemäß bevorzugte Mittel zum Färben und/oder Aufhellen keratinischer Fasern sind dadurch gekennzeichnet, daß sie mindestens einen direktziehenden Farbstoff enthalten, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Eine Färbung keratinischer Fasern kann auch mittels Farbstoffen erfolgen, die in einer oxidativ katalysierten Reaktion von C,H-aciden Verbindungen mit reaktiven Carbonylverbindungen gebildet werden.

In einer weiteren Ausführungsform enthält das erfindungsgemäße Mittel daher eine Kombination aus Komponente
A Verbindungen, die eine reaktive Carbonylgruppe enthalten mit Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden.

Als Oxofarbstoffvorprodukte kommt bevorzugt eine Kombination aus
- mindestens einer Verbindung, die mindestens eine reaktive Carbonylgruppe enthält
   (Komponente (Oxo 1))
   mit mindestens einer Verbindung (Komponente Oxo2)
- Verbindungen, ausgewählt aus
   (Oxo2a) CH-aciden Verbindungen
   und/oder aus
   (Oxo2b) Verbindungen mit primärer oder sekundärer Aminogruppe oder Hydroxygruppe,
   ausgewählt aus mindestens einer Verbindung der Gruppe, die gebildet wird aus primären oder sekundären aromatischen Aminen, stickstoffhaltigen heterozyklischen
   Verbindungen und aromatischen Hydroxyverbindungen
   zum Einsatz.

Diese Verbindungen werden im Prioritätsdokument detailliert beschrieben.

Die erfindungsgemäßen Mittel zum Färben und/oder Aufhellen keratinischer Fasern können auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden.

Ein weiterer Gegenstand der vorliegenden Erfindung ist daher ein erfindungsgemäßes Mittel zum Färben und/oder Aufhellen keratinischer Fasern, insbesondere menschlicher Haare, welches unmittelbar vor dem Aufbringen auf das Haar aus einer fließfähigen Zubereitung A, einer Oxidationsmittelzubereitung B, enthaltend mindestens ein Oxidationsmittel, ausgewählt aus Wasserstoffperoxid und dessen Anlagerungsverbindungen an feste Träger und gegebenenfalls einer Zubereitung C, die miteinander zu einem Färbe- und/oder Aufhellansatz vermischt werden, erhalten wird. Eine oder mehrere dieser Zubereitungen A, B und/oder C enthalten demnach *trans-*4-*tert*-Butyl-Cyclohexanol. Oxidationsfarbstoffvorprodukte und/oder direktziehende Farbstoffe sind dabei zumeist in der Zubereitung A enthalten.

Die Mischung der Zubereitungen A, B und C vor der Anwendung führt zu einer Anwendungsmischung, die *trans*-4-*tert*-Butyl-Cyclohexanol sowie Oxidationsmittel und/oder Farbstoffe und/oder Farbstoffvorprodukte enthält.

Besonders bevorzugt werden die Zubereitungen A und B miteinander in einem Gewichtsverhältnis von 4:1 bis 1:4, vorzugsweise 3:1 bis 1:3, weiter bevorzugt 2:1 bis 1:2 und insbesondere 1:2 bis 1:1,5 vermischt. Bevorzugte Mischungsverhältnisse von (A+B) zu C liegen im Bereich von 120:1 bis 1:1, vorzugsweise von 100:1 bis 1,5:1, weiter bevorzugt von 80:1 bis 2:1 und insbesondere von 50:1 bis 3:1.

Vorzugsweise werden die erfindungsgemäßen Mittel als Aufhellmittel (nachfolgend auch als Blondiermittel bezeichnet) und/oder als Färbemittel bereitgestellt. Mittel, welche gleichzeitig färbend und aufhellend wirken, werden auch als aufhellende Färbemittel bezeichnet.

Im Falle der Mehrkomponentenmittel gemäß der zweiten Ausführungsform der vorliegenden Erfindung sind die Oxidationsfarbstoffvorprodukte vom Entwickler- und Kupplertyp vorzugsweise in der Zubereitung A enthalten.

Die Oxidationsmittelzubereitung B enthält mindestens ein Oxidationsmittel, ausgewählt aus Wasserstoffperoxid und dessen Anlagerungsverbindungen an feste Träger. Als erfindungsgemäßes Oxidationsmittel wird bevorzugt Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid und Melaminperoxid, eingesetzt.

Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12- prozentige Lösungen in Wasser verwendet. Erfindungsgemäß bevorzugte Mittel zum Färben und/oder Aufhellen keratinischer Fasern sind dadurch gekennzeichnet, daß sie 0,5 bis 15 Gew.-%, vorzugsweise 1 bis 12,5 Gew.-%, besonders bevorzugt 1 bis 10 Gew.-% und insbesondere 1 bis 8 Gew.-% Wasserstoffperoxid (berechnet als 100%iges H₂O₂) enthalten.

Ein bevorzugtes erfindungsgemäßes Mittel enthält neben dem Oxidationsmittel Wasserstoffperoxid mindestens ein kationisches Acylpyridiniumderivat der Formel (I), 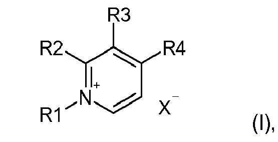 worin
- R1: für eine C₁-C₆-Alkylgruppe, eine C₂-C₆-Alkenylgruppe, eine C₂-C₆-Hydroxy-alkylgruppe, eine C₁-C₆-Alkoxy-C₂-C₆-alkylgruppe, eine Carboxy-C₂-C₆-alkylgruppe, eine Aryl-C₁-C₆-alkylgruppe, eine Heteroaryl-C₁-C₆-alkylgruppe, eine Arylgruppe oder eine Heteroarylgruppe steht,
- R2, R3 und R4: jeweils unabhängig voneinander für Wasserstoff, eine C₁-C₆-Alkylgruppe, ein Halogenatom oder eine C₁-C₆-Acylgruppe steht, mit der Massgabe, dass zumindest einer der Reste R2, R3 und R4 für eine C₁-C₆-Acylgruppe steht,
- X⁻: für ein physiologisch verträgliches Anion steht,

Beispiele für die als Substituenten der Verbindungen der Formel (I) genannten werden nachfolgend, aber nicht beschränkend genannt: Beispiele für C₁-C₆-Alkylreste sind die Gruppen -CH₃, - CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃. Beispiele für eine C₂-C₆-Alkenylgruppe sind eine Prop-2-enylgruppe (Allylgruppe), eine 2-Methyl-prop-2-enylgruppe, eine But-3-enylgruppe, eine But-2-enylgruppe, eine Pent-4-enylgruppe oder eine Pent-3-enylgruppe, wobei die Prop-2-enylgruppe bevorzugt ist. Beispiele für eine C₂-C₆-Hydroxyalkylgruppe sind -CH₂CH₂OH, -CH₂CH₂CH₂OH, -CH₂CH(OH)CH₃ und - CH₂CH₂CH₂CH₂OH, wobei die Gruppe -CH₂CH₂OH bevorzugt ist. Beispiele für C₁-C₆-Alkoxy-C₂-C₆-alkylgruppen sind die Gruppen -CH₂CH₂OCH₃, -CH₂CH₂CH₂OCH₃, -CH₂CH₂OCH₂CH₃, - CH₂CH₂CH₂OCH₂CH₃, -CH₂CH₂OCH(CH₃)₂, -CH₂CH₂CH₂OCH(CH₃)₂. Beispiele für eine Carboxy-C₁-C₆-alkylgruppe sind die Carboxymethylgruppe, die 2-Carboxyethylgruppe oder die 3-Carboxypropylgruppe. Beispiele für Aryl-C₁-C₆-alkylgruppen sind die Benzylgruppe und die 2-Phenylethylgruppe. Beispiele für eine Heteroaryl-C₁-C₆-alkylgruppe sind die Pyridin-2-ylmethylgruppe, die Pyridin-3-ylmethylgruppe, die Pyridin-4-ylmethylgruppe, die Pyrimidin-2-ylmethylgruppe, die Pyrrol-1-ylmethylgruppe, die Pyrrol-1-ylethylgruppe, die Pyrazol-1-ylmethylgruppe oder die Pyrazol-1-ylethylgruppe. Beispiele für eine Arylgruppe sind die Phenylgruppe, die 1-Naphthylgruppe oder die 2-Naphthylgruppe. Beispiele für eine Heteroarylgruppe sind die Pyridin-2-ylgruppe, die Pyridin-3-ylgruppe, die Pyridin-4-ylgruppe, die Pyrimidin-2-ylgruppe, die Pyrrol-1-ylgruppe, die Pyrrol-2-ylgruppe, die Pyrazol-1-ylgruppe, die Pyrazol-3-ylgruppe oder die Pyrazol-4-ylgruppe. Beispiele einer C₁-C₆-Acylgruppe sind Acetyl (1-Oxo-ethyl), 1-Oxo-propyl, 1-Oxo-butyl, 1-Oxo-Pentyl, 1-Oxo-2,2-dimethylpropyl und 1-Oxo-hexyl.

In einer Ausführungsform der vorliegenden Erfindung sind solche Verbindungen gemäß Formel (I) bevorzugt, bei welchen der Rest R1 der allgemeinen Struktur (I) für eine C₁-C₆-Alkylgruppe, für eine C₂-C₆-Alkenylgruppe oder für eine C₂-C₆-Hydroxyalkylgruppe steht. Es ist erfindungsgemäß bevorzugt, wenn der Rest R1 für eine C₁-C₆-Alkylgruppe, bevorzugt für Methyl, Ethyl, n-Propyl oder Isopropyl und insbesondere bevorzugt für Methyl, steht.

Es hat sich herausgestellt, dass die Acylpyridiniumderivate gemäß Formel (I) erfindungsgemäß besonders vorteilhafte Eigenschaften besitzen, wenn sie die Acyl-Gruppe entweder in 2- oder 4-Position am Pyridin-Ring tragen. Bevorzugte Verbindungen der Formel (I) sind weiterhin solche Verbindungen, bei denen entweder der Rest R2 oder der Rest R4 für eine C₁-C₆-Acylgruppe, bevorzugt für eine Acetylgruppe, steht. Es ist weiterhin bevorzugt, wenn einer der Reste R2 oder R4 für eine Acetylgruppe steht, während der andere dieser Reste sowie der Rest R3 jeweils für Wasserstoff stehen. Eine weitere Ausführungsform der vorliegenden Erfindung ist daher dadurch gekennzeichnet, dass das Mittel als Acylpyridiniumderivat gemäß Formel (I) mindestens ein 2-Acetylpyridiniumderivat und/oder 4-Acetylpyridiniumderivat enthält. Geeignete Acetylpyridiniumderivate sind dabei insbesondere die physiologisch verträglichen Salze, die als Kation ein Acetylpyridiniumderivat, ausgewählt aus 4-Acetyl-1-methylpyridinium, 4-Acetyl-1-allylpyridinium, 4-Acetyl-1-(2-hydroxyethyl)pyridinium, 2-Acetyl-1-methylpyridinium, 2-Acetyl-1-allylpyridinium und 2-Acetyl-1-(2-hydroxyethyl)pyridinium, enthalten.

Es ist bevorzugt, wenn das Anion X- gemäß Formel (I) ausgewählt wird aus Halogenid, insbesondere Chlorid, Bromid und Iodid, Benzolsulfonat, p-Toluolsulfonat, C₁-C₄-Alkylsulfonat, Trifluormethansulfonat, Acetat, Triflluoracetat, Perchlorat, ½ Sulfat, Hydrogensulfat, Tetrafluorborat, Hexafluorphosphat oder Tetrachlorzinkat. Erfindungsgemäß vorteilhaft ist es, wenn das physiologisch verträgliche Anion X- für ein Halogenidion (insbesondere Chlorid oder Bromid), Hydrogensulfat, p-Toluolsulfonat, Benzolsulfonat oder Acetat steht.

Insbesondere sind solche Mittel erfindungsgemäß bevorzugt, die dadurch gekennzeichnet sind, dass das Acylpyridiniumderivat gemäß Formel (I) ausgewählt ist aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridinium-hydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumhydrogensulfat und 2-Acetyl-1-allylpyridiniumacetat. Erfindungsgemäß bevorzugte Mittel sind dadurch gekennzeichnet, dass sie als das Acylpyridiniumderivat gemäß Formel (I) eine Verbindung, ausgewählt aus 4-Acetyl-1-methylpyridinium-p-toluolsulfonat und/oder 2-Acetyl-1-methylpyridinium-p-toluolsulfonat, insbesondere 4-Acetyl-1-methylpyridinium-p-toluolsulfonat, enthalten.

Eine Ausführungsform der vorliegenden Erfindung ist dabei dadurch gekennzeichnet, dass im erfindungsgemäßen Mittel die Acylpyridiniumderivate der Formel (I), insbesondere insbesondere 4-Acetyl-1-methylpyridinium-p-toluolsulfonat, in einer Menge von 0,1 bis 10 Gew.-%, insbesondere von 0,2 bis 4 Gew.-%, jeweils bezogen das Gesamtgewicht des Mittels, enthalten sind.

Die Oxidationsmittelzubereitung B ist vorzugsweise eine wäßrige, fließfähige Oxidationsmittelzubereitung. Demnach sind bevorzugte erfindungsgemäße Mittel dadurch gekennzeichnet, daß die fließfähige Oxidationsmittelzubereitung B - bezogen auf ihr Gewicht-40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält. Vorzugsweise wird der fließfähigen Oxidationsmittelzubereitung B ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind. Diese Stoffe werden weiter unten ausführlich beschrieben.

Die Oxidationsmittelzubereitung kann weitere Oxidationsmittel enthalten, die auch als "Booster" bezeichnet werden. Die Auswahl dieser weiteren Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten. Hier sind erfindungsgemäße Mittel bevorzugt, bei denen die fließfähige Oxidationsmittelzubereitung B zusätzlich mindestens eine Peroxoverbindung enthält, die vorzugsweise ausgewählt ist aus Ammonium- und Alkalimetallpersulfaten und -peroxidisulfaten, wobei bevorzugte Mittel mindestens 2 verschiedene Peroxidisulfate enthalten.

Bei einer Anwendung von Oxidationsmitteln wird das eigentliche Färbemittel wie weiter oben erwähnt, zweckmäßigerweise unmittelbar vor der Anwendung durch Mischung der Zubereitung des Oxidationsmittels B mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Für eine Farbveränderung mittels Aufhellung bzw. Bleichung des Substrats, beispielsweise der Haare, wird bevorzugt in den erfindungsgemäßen kosmetischen Mitteln neben den Oxidationsmitteln zusätzlich mindestens ein Bleichverstärker eingesetzt.

Bleichverstärker werden bevorzugt zur Steigerung der Bleichwirkung des Oxidationsmittels, insbesondere des Wasserstoffperoxids, eingesetzt. Geeignete Bleichverstärker sind (BV-i) Verbindungen, die unter Perhydrolysebedingungen aliphatische Peroxocarbonsäuren
und/oder gegebenenfalls substituierte Perbenzoesäure ergeben, und/oder
(BV-ii) Carbonatsalze und/oder Hydrogencarbonatsalze, und/oder
(BV-iii) organische Carbonate, und/oder
(BV-iv) Carbonsäuren, und/oder
(BV-v) Peroxoverbindungen.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die sauerstoff- und/oder stichstoffgebundene Acylgruppen mit der genannten Anzahl an Kohlenstoffatomen und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Die Carbonat- bzw. Hydrogencarbonatsalze werden bevorzugt ausgewählt aus mindestens einer Verbindung der Gruppe bestehend aus Ammonium-, Alkali- (insbesondere Natrium- und Kalium-), sowie Erdalkali- (insbesondere Calcium-), -carbonatsalzen und -hydrogencarbonatsalzen.

Besonders bevorzugte Carbonat-, bzw. Hydrogencarbonatsalze sind

Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Bleichverstärker verwendet werden.

Bevorzugt nutzbare organische Carbonate werden ausgewählt aus mindestens einer Verbindung der Gruppe der Kohlensäuremonoester und/oder aus mindestens einer Verbindung der Gruppe der Kohlensäuremonoamide.

Bevorzugt verwendbare Kohlensäuremonoester sind die Kohlensäuremonoester der Formel (BV-1),

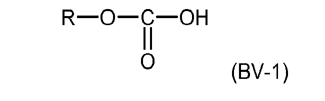

in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (BV-1) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugt verwendete Zusammensetzungen sind dadurch gekennzeichnet, daß der Rest R in Formel (BV-1) ausgewählt ist aus Methyl-, Ethyl-, n-Propyl-, isoPropyl-, n-Butyl-, iso-Butyl-, *tert*-Butyl- sowie Hydroxymethyl- und Hydroxyethyl-Resten. Bevorzugte Kohlensäuremonoamide sind die Verbindungen der Formel (BV-2),

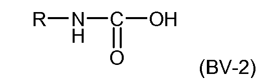

in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (BV-2) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Bleichverstärker der Formel (BV-2) sind dadurch gekennzeichnet, daß der Rest R in Formel (BV-2) ausgewählt ist aus Methyl-, Ethyl-, n-Propyl-, isoPropyl-, n-Butyl-, iso-Butyl-, *tert*-Butyl- sowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkalimetall-, Ammonium-, Erdalkalimetall- oder Aluminiumsalzes und insbesondere in Form seines Natriumsalzes, vorliegen.

Als bleichverstärkende Carbonsäure kann in den erfindungsgemäßen Zusammensetzungen bevorzugt mindestens eine Verbindung aus der Gruppe, bestehend aus Essigsäure, Milchsäure, Weinsäure, Zitronensäure, Salicylsäure und ortho-Phthalsäure, enthalten sein.

Bleichverstärker sind bevorzugt Peroxoverbindungen, insbesondere anorganische Peroxoverbindungen. Unter die erfindungsgemäß bleichverstärkenden Peroxoverbindungen fallen keine Anlagerungsprodukte von Wasserstoffperoxid an andere Komponenten und auch nicht Wasserstoffperoxid selbst. Die Auswahl der Peroxoverbindungen unterliegt darüber hinaus keinen Beschränkungen. Bevorzugte Peroxoverbindungen sind Peroxidisulfatsalze, Persulfatsalze, Peroxidiphosphatsalze (insbesondere Ammoniumperoxidisulfat, Kaliumperoxidisulfat, Natriumperoxidisulfat, Ammoniumpersulfat, Kaliumpersulfat, Natriumpersulfat, Kaliumperoxidiphosphat) und Peroxide (wie Bariumperoxid und Magnesiumperoxid). Unter diesen Peroxoverbindungen, die auch in Kombination eingesetzt werden können, sind erfindungsgemäß die Peroxidisulfate, insbesondere Ammoniumperoxidisulfat, bevorzugt.

Die Bleichverstärker sind in den erfindungsgemäßen, kosmetischen Mitteln bevorzugt in Mengen von 5 bis 30 Gew.-%, insbesondere in Mengen von 8 bis 20 Gew.-%, jeweils bezogen auf das Gewicht des anwendungsbereiten Mittels, enthalten.

Zusätzlich können die erfindungsgemäßen Mittel weitere Inhaltsstoffe enthalten. Ein Einsatz bestimmter Metallionen oder-komplexe kann beispielsweise bevorzugt sein, um intensive Färbungen zu erhalten. Hier sind erfindungsgemäße Mittel bevorzugt, die zusätzlich Cu-, Fe-, Mn-, Ru-Ionen oder Komplexe dieser Ionen enthalten.

Bevorzugte erfindungsgemäße Haarfärbe- und - aufhellungsmittel enthalten 0,0001 bis 2,5 Gew.-%, vorzugsweise 0,001 bis 1 Gew.-%, bezogen auf die Gesamtzusammensetzung des Mittels, mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Kaliumjodid, Natriumjodid, Lithiumchlorid, Kaliumdichromat, Magnesiumacetat, Calciumchlorid, Calciumnitrat, Bariumnitrat, Mangandioxid (MnO₂) und/oder Hydrochinon.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprdukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Im Rahmen einer bevorzugten Ausführungsform handelt es sich bei den erfindungsgemäßen Mittel um dauerhaft formverändernde Mittel. Diese Mittel bestehen üblicherweise aus zwei beziehungsweise drei Zubereitungen, die nacheinander auf die Fasern aufgebracht werden. Im Weiteren werden folgende Bezeichnungen verwendet:
- "Wellmittel" für die wässrige Zubereitung der keratinreduzierenden Substanz,
- "Zwischenspülung" für die erste Spülung und
- "Fixiermittel" für die wässrige Zubereitung des Oxidationsmittels.

Obwohl das *trans*-4-*tert*-Butyl-Cyclohexanol prinzipiell in jeder der genannten Zubereitungen eingesetzt werden kann, hat es sich als erfindungsgemäß besonders bevorzugt erwiesen, wenn es im Wellmittel enthalten ist.

Die erfindungsgemäßen Wellmittel enthalten zwingend mindestens ein keratinreduzierende Substanz als formverändernde Komponente, vorzugsweise Mercaptane. Solche Verbindungen sind beispielsweise Thioglykolsäure, Thiomilchsäure, Thioäpfelsäure, Mercaptoethansulfonsäure sowie deren Salze und Ester, Cysteamin, Cystein, Bunte Salze und Salze der schwefligen Säure. Bevorzugt geeignet als formverändernde Komponenten sind die Alkali- oder Ammoniumsalze der Thioglykolsäure und/oder der Thiomilchsäure sowie die freien Säuren. Diese werden in den Wellmitteln bevorzugt in Konzentrationen von 0,5 bis 1,0 mol/kg bei einem pH-Wert von 5 bis 12, insbesondere von 7 bis 9,5, eingesetzt. Zur Einstellung dieses pH-Wertes enthalten die erfindungsgemäßen Wellmittel üblicherweise Alkalisierungsmittel wie Ammoniak, Alkali- und Ammoniumcarbonate und -hydrogencarbonate oder organische Amine wie Monoethanolamin. Weiterhin können die erfindungsgemäßen Welllotionen wellkraftverstärkende Komponenten enthalten, bevorzugt ausgewählt aus heterocyclischen Verbindungen wie Imidazol, Pyrrolidin, Piperidin, Dioxolan, Dioxan, Morpholin, Piperazin sowie Derivate dieser Verbindungen, insbesondere 1-Methylimidazol, 2-Methylimidazol, 4(5)-Methylimidazol, 1,2-Dimethylimidazol, 2-Ethylimidazol, 2-Isopropylimidazol, N-Methylpyrrolidon, 1-Methylpiperidin, 4-Methylpiperidin, 2-Ethylpiperidin, 4-Methylmorpholin, 4-(2-Hydroxyethyl)morpholin, 1-Ethylpiperazin, 1-(2-Hydroxyethyl)piperazin, 1-(2-Aminoethyl)piperazin, Biotin, Hydantoin und Benzimidazol, wobei Imidazol besonders bevorzugt ist; Aminosäuren wie insbesondere Arginin, Citrullin, Histidin, Ornithin, Lysin, Oligopeptiden aus durchschnittlich 2-3 Aminosäuren, die einen hohen Anteil (> 50 %, insbesondere > 70 %) an den genannten Aminosäuren haben, sowie deren Salze, bevorzugt Arginin sowie dessen Salze und Arginin-reiche Oligopeptide; Diole wie beispielsweise 2-Ethyl-1,3-hexandiol, 1,3-Butandiol, 1,4-Butandiol, 1,2-Propandiol, 1,3-Propandiol, Neopentylglykol und Ethylenglykol, wobei 1,3-Diole, insbesondere 2-Ethyl-1,3-hexandiol und 1,3-Butandiol besonders bevorzugt sind. Die wellkraftverstärkenden Verbindungen können in den erfindungsgemäßen Welllotionen in Mengen von 0,5 bis 5 Gew.-%, bezogen auf die gesamte Welllotion, enthalten sein. Mengen von 1 bis 4 Gew.-%, im Falle der Diole von 0,5 bis 3 Gew.-%, haben sich als ausreichend erwiesen, weshalb diese Mengen besonders bevorzugt sind.

Zwingender Bestandteil der erfindungsgemäßen Fixiermittel sind Oxidationsmittel, z. B. Natriumbromat, Kaliumbromat, Wasserstoffperoxid, und die zur Stabilisierung wässriger Wasserstoffperoxidzubereitungen üblichen Stabilisatoren. Erfindungsgemäß einsetzbare wässriger H₂O₂-Zubereitungen enthalten etwa 0,5 bis 15 Gew.-%, gebrauchsfertig in der Regel etwa 0,5 bis 3 Gew.-%, Wasserstoffperoxid. Der pH-Wert solcher wässriger H₂O₂-Zubereitungen liegt bevorzugt bei 2 bis 6, insbesondere 2 bis 4 und wird durch anorganische Säuren, bevorzugt Phosphorsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Gleichfalls geeignet sind Fixiermittel auf enzymatischer Basis (z. B. Peroxidasen), die keine oder nur geringe Mengen an Oxidationsmitteln, insbesondere Wasserstoffperoxid, enthalten.

Die erfindungsgemäßen Wellmittel beziehungsweise Fixiermittel sind üblicherweise einphasig formuliert, eingeschlossen von diesem Begriff sind dabei Systeme, die eine kontinuierliche Phase aufweisen, wie beispielsweise reine ÖI-in-Wasser- oder Wasser-in-ÖI-Emulsionen. Es hat sich gezeigt, das erfindungsgemäß auch Zwei- und Mehrphasensysteme bevorzugt sind. Dies sind Systeme, bei denen mindestens zwei separate, kontinuierliche Phasen vorliegen.

Weiterhin hat es sich als vorteilhaft erweisen, wenn die Oxidationsmittelzubereitungen aus Oxidationsfärbemitteln, Aufhellmitteln und/oder Fixiermitteln von formverändernden Mitteln mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP), Diethylentriaminpenta-(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Die erfindungsgemäßen Mittel enthalten weitere Hilfs- und Zusatzstoffe.

Es hat sich herausgestellt, dass durch den Zusatz eines wasserlöslichen, organischen Lösungsmittels in das erfindungsgemäße Mittel die Haarschädigung weiter verringert.

Eine bevorzugte Ausführungsform der vorliegenden Erfindung ist daher dadurch gegeben, dass das Mittel weiterhin mindestens ein wasserlösliches, organisches Lösungsmittel enthält. Die organischen Lösungsmittel sind bei Raumtemperatur unter Normaldruck flüssig. Unter wasserlöslich wird hierin die Mischbarkeit in jedem Verhältnis unter Normalbedingungen verstanden. Bevorzugte organische Lösungsmittel sind dabei C₁-C₆-Alkohole, die gegebenenfalls weitere funktionelle Gruppen zur Verbesserung der Wasserlöslichkeit tragen, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether. Bevorzugte wasserlösliche, organische Lösungsmittel enthalten mindestens zwei Hydroxylgruppen. Bevorzugt sind diese ausgewählt aus der Gruppe, die gebildet wird aus 1,2-Ethandiol, 1,2-Propandiol (1,2-Propylenglykol), 1,3-Propandiol, 1,2-Butandiol, 1,3-Butandiol, 1,4-Butandiol, 2,3-Butandiol, 1,6-Hexandiol, 2-(2-Hydroxyethoxy)ethanol und Glycerin. Besonders bevorzugt sind dabei Glycerin sowie Butylenglycol (1,2-Butandiol, 1,3-Butandiol, 1,4-Butandiol und 2,3-Butandiol).

Bevorzugt enthält das erfindungsgemäße Mittel wasserlösliche, organische Lösungsmittel zu 0,01 bis 10 Gew.-%, bevorzugt zu 0,05 bis 5 Gew.-%, jeweils bezogen auf das Gesamtgewicht des anwendungsbereiten Mittels.

Es hat sich weiterhin herausgestellt, dass sich die Hautirritation durch Haarfärbe- und/oder Haarwellmittel durch den Zusatz bestimmter Pflegestoffe zusätzlich unerwartet deutlich reduzieren lässt. Als erfindungsgemäß besonders vorteilhaften zusätzlichen Pflegestoff hat sich dabei der Zellschutzstoff Ectoin ((4S)-1,4,5,6-Tetrahydro-2-methyl-pyrimidine-4-carboxylic acid; INCl-Bezeichnung: Ectoin) erwiesen. Eine weiterhin bevorzugte Ausführungsform der vorliegenden Erfindung ist daher dadurch gegeben, dass das Mittel zusätzlich Ectoin enthält. Bevorzugt enthält das erfindungsgemäße Mittel Ectoin zu 0,0001 bis 3,0 Gew.-%, bevorzugt zu 0,001 bis 1,0 Gew.-%, jeweils bezogen auf das Gesamtgewicht des anwendungsbereiten Mittels.

In einer bevorzugten Ausführungsform der Einfindung enthalten die Haarbehandlungsmittel - bezogen auf ihr Gewicht - neben der erfindungsgemäßen Wirkstoffkombination weiterhin 0,01 bis 2 Gew.-%, bevorzugt 0,05 bis 1,5 Gew.-% und insbesondere 0,1 bis 1 Gew.-% D-Panthenol.

In einer weiteren bevorzugten Ausführungsform der Einfindung enthalten die Haarbehandlungsmittel - bezogen auf ihr Gewicht - neben der erfindungsgemäßen Wirkstoffkombination 0,01 bis 2

Gew.-%, bevorzugt 0,05 bis 1,5 Gew.-% und insbesondere 0,1 bis 1 Gew.-% Glycin, Serin und/oder Arginin.

Erfindungsgemäß ganz besonders bevorzugt aufgrund ihrer haarkräftigenden Wirkung sowie ihrer unterstützenden Wirkung in Bezug auf die Haarfülle sind die Extrakte aus Grünem Tee, Kamille, Mandel und Moringa. Insbesondere bevorzugt ist erfindungsgemäß ein Moringa-Extrakt, beispielsweise ein unter dem Handelsnamen Puricare^{®} LS 9658 oder Moringa Oleifera^{®} vertriebenes Produkt.

In einer weiteren bevorzugten Ausführungsform der Einfindung enthalten die Haarreinigungs- und/oder -konditioniermittel - bezogen auf ihr Gewicht - neben der erfindungsgemäßen Wirkstoffkombination weiterhin 0,01 bis 2 Gew.-%, bevorzugt 0,05 bis 1,5 Gew.-% und insbesondere 0,1 bis 1 Gew.-% Moringa-Extrakt.

Erfindungsgemäß bevorzugte Mittel enthalten einen sebumsorbierenden Wirkstoff, vorzugsweise ausgewählt aus anorganischen und organischen kosmetischen Adsorbentien mit mittleren Partikeldurchmessern von 0,1-100 µm, bevorzugt 0,5-50 µm, besonders bevorzugt 5-30 µm und außerordentlich bevorzugt 10-25 µm. Besonders bevorzugte anorganische Adsorbentien sind ausgewählt aus Kieselsäuren, insbesondere Aerosil^{®}-Typen, Kieselgelen, Siliciumdioxid, Tonen und Schichtsilicaten, insbesondere Bentoniten oder Kaolin, Magnesiumaluminiumsilikaten, insbesondere Talkum, und Bornitrid, sowie Mischungen der genannten Substanzen. Besonders bevorzugte organische Adsorbentien sind ausgewählt aus Stärken und Stärkederivaten, die chemisch und/oder physikalisch modifiziert sein können, insbesondere modifizierten Stärkederivaten vom Typ DRY FLO^{®} der National Starch and Chemical Company, Cellulosepulvern, Lactoglobulinderivaten, insbesondere Natrium-C₈₋₁₆-Isoalkylsuccinyllactoglobulinsulfonat, z. B. als Handelsprodukt Biopol^{®} OE von Brooks Industries erhältlich, Polymerpulvern aus Polyolefinen, insbesondere Polyethylen-Pulvern und Polypropylen-Pulvern, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, wobei die Polymerpulver in einer besonders bevorzugten Ausführungsform der Erfindung vernetzt sein können, sowie Mischungen der genannten Substanzen.

Bevorzugte erfindungsgemäße Mittel enthalten als Pflegewirkstoff mindestens einen Extrakt aus der Wurzel von Boerhavia diffusa. Bedingt durch die Schutzwirkung bei Anwendung des erfindungsgemäßen Mittels kann die Hautverträglichkeit hierdurch deutlich verbessert werden.

Es hat sich gezeigt, dass die Extrakte aus der Wurzel von Boerhavia diffusa dazu geeignet sind, in der Haut, insbesondere der Kopfhaut, die nucleare Translocation und Aktivierung von des Transkriptionsfaktors NF-κB (nuclearer Faktor κB / (kappa)B) sowie die Produktion von entzündungsverstärkenden Prostaglandinen, insbesondere PGE2, in den Keratinocyten zu reduzieren und damit insgesamt die Hautverträglichkeit erfindungsgemäßer Mittel zu verbessern. Darüber hinaus begünstigen die Wirkstoffe aus dem Extrakt der Wurzeln von Boerhavia diffusa die Bildung des Hormons α-MSH (alpha-Melanocyten-stimulierendes Hormon), welches eine entscheidende Rolle bei der Regulation und Verringerung von entzündlichen Empfindungen, insbesondere des Juckreizes, spielt. Dadurch wird insgesamt die Toleranzschwelle der Haut, auf irritierende Substanzen mit Reizung und Entzündungen zu reagieren, herabgesenkt. Somit werden sowohl Entzündungspotential wie auch Schmerzempfindung der Kopfhaut reduziert. Außerdem kann sich angegriffene, empfindliche Haut beruhigen und so potentiell allergienauslösende Substanzen besser tolerieren. Insgesamt führt die Verwendung von Extrakten aus der Wurzel von Boerhavia diffusa in Mittel zur Farb- und/oder Formveränderung von menschlichen Haaren zu einer Verbesserung der Hautverträglichkeit und zu einer Verringerung an Irritationen auf der Kopfhaut. Der Extrakt aus der Wurzel der Boerhavia diffusa ist dabei reich an unterschiedlichsten Polyphenolen und Hydroxybenzoesäuren, welche für die Wirksamkeit des Pflanzenextrakts verantwortlich sind. Diese Extrakte lassen sich herstellen, indem die gemahlenen, pulverisierten Wurzeln von Boerhavia diffusa mit einer Mischung aus Wasser und Alkohol, bevorzugt Butylenglycol, aufgenommen werden. Die Wasser-Alkohol-Mischung besitzt dabei bevorzugt ein Mischungsverhältnis von 3:1 bis 1:3 Volumenteilen, besonders bevorzugt von 1:1. Der unlösliche Anteil wird abgetrennt und der lösliche Anteil durch thermische Einwirkung inaktiviert, anschließend filtriert und dann sterilisiert. Als hauptsächliche Wirkstoffe des Extrakts gelten die darin enthaltenen Polyphenole. Bevorzugte Extrakte enthalten ca. 0,05 bis 5 g/L an Polyphenolen, insbesondere 0,20 bis 0,55 g/L. Die Polyphenole setzen sich dabei überwiegend aus Hydroxybenzoesäuren (ca. 50 bis 70 Gew.-% aller enthaltenen Polyphenole) sowie aus Flavonoiden (24 bis 33 Gew.-%) und untergeordnet aus Hydroxyzimtsäuren (1 bis 5 Gew.-%) zusammen.

Ein erfindungsgemäß besonders geeigneter Extrakt aus den Wurzeln von Boerhavia diffusa ist das Handelsprodukt Mediacalm^{®}, welches von der Firma Silab vertrieben wird und welches nach INCl mit Aqua, Butylene glycol und Boerhavia diffusa root extract bezeichnet wird.

In einer Ausführungsform sind erfindungsgemäße Mittel dadurch gekennzeichnet, dass sie den Extrakt aus Wurzeln von Boerhavia diffusa in einer Menge von 0,00001 bis 10,0 Gew.-%, insbesondere von 0,0001 bis 8,0 Gew.-%, jeweils bezogen auf das Gesamtgewicht des anwendungsbereiten Mittels, enthalten. Ein erfindungsgemäß besonders bevorzugtes Mittel ist dadurch gekennzeichnet, dass es den Extrakt aus Wurzeln von Boerhavia diffusa in einer Menge von 0,0001 bis 5 Gew.-%, insbesondere von 0,001 bis 1,0 Gew.-%, jeweils bezogen auf das Gesamtgewicht des anwendungsbereiten Mittels, enthält. Diese Pflegestoffe können einzeln, bevorzugt jedoch in Kombinationen untereinander im erfindungsgemäßen Mittel eingesetzt werden. Eine weitere Ausführungsform der vorliegenden Erfindung ist daher dadurch gekennzeichnet, dass das Mittel als zusätzlichen Haarpflegestoff mindestens einen Pflegestoff, ausgewählt aus der Gruppe, gebildet aus D-Panthenol, Glycin, Serin, Arginin, Moringa Oleifera und/oder Ectoin, enthält. Vorzugsweise stellen die anwendungsbereiten Mittel fließfähige Zubereitungen dar. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und weiteren nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe.

Bevorzugte anionische Tenside sind Alkylsulfate, Alkylethersulfate, jeweils mit 10 bis 18 C-Atomen in der Alkylgruppe, und polyalkoxylierte Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Ein bevorzugtes zwitterionisches Tensid ist das unter der INCI-Bezeichnung Cocamidopropyl Betaine bekannte Fettsäureamid-Derivat.

In einer weiteren, bevorzugten Ausführungsform der vorliegenden Erfindung enthält das Mittel weiterhin mindestens ein amphoteres Tensid. Besonders bevorzugte amphotere Tenside werden unter der INCI-Bezeichnung Disodium Cocoamphodipropionate mit den Handelsnamen Miranol C2M SF conc. (Rhodia), Amphoterge K-2 (Lonza) und Monateric CEM-38 (Unichema) und Bezeichnung Disodium Cocoamphodiacetate mit den Handelsnamen Dehyton (Cognis), Miranol C2M (Rhodia) und Ampholak XCO 30 (Akzo Nobel) vermarktet.

Der Zusatz im erfindungsgemäßen Mittel bewirkt eine hervorragende Avivage, insbesondere eine verbesserte Nasskämmbarkeit.

Durch den Zusatz oberflächenaktiver Verbindungen lässt sich die Effektivität der erfindungsgemäßen Mittel weiter verbessern. Als besonders vorteilhaft haben sich insbesondere weitere oberflächenaktiver Verbindungen vom Typ nichtionischer Tenside herausgestellt. Solche Verbindungen sind beispielsweise Anlagerungsprodukte von Polyethylenoxid an Fettalkohole, wobei Anlagerungsprodukte von Polyethylenoxid an Fettalkohole besonders bevorzugt sind, die einen durchschnittlichen Ethoxylierungsgrad von 10 bis 45, insbesondere von 12 bis 30 aufweisen, wie beispielsweise Steareth-20, Coceth-15, Oleth-20 oder auch Ceteareth-30 sowie Alkylpolyglykoside entsprechend der allgemeinen Formel RO-(Z)ₓ, wobei R für Alkyl, Z für Zucker sowie x für die Anzahl der Zuckereinheiten steht. Erfindungsgemäß bevorzugte Mittel enthalten als nichtionisches Tensid das Anlagerungsprodukt von Cocos-Fettalkohol an (Poly-)glucose, welches unter der INCI-Bezeichnung Coco-Glucoside bekannt ist.

Die anionischen, zwitterionischen, amphoteren und weiteren nichtionischen Tenside werden in bevorzugt in einer Gesamtmenge von 0,1 bis 45 Gew.%, bevorzugt 1 bis 30 Gew.% und ganz besonders bevorzugt von 1 bis 20 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Es hat sich erfindungsgemäß als vorteilhaft erwiesen, wenn Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Bevorzugt handelt es sich bei dem Verdickungsmittel um ein anionisches, synthetisches Polymer. Bevorzugte anionische Polymere sind Acrylsäure- und/oder Methacrylsäure-Polymerisate oder-Copolymerisate, die in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten sind. Weiterhin bevorzugt handelt es sich bei dem Verdickungsmittel um ein kationisches synthetisches Polymer. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen. Solche Polymere können auch als Copolymere mit nichtionogene Monomereinheiten bevorzugt Acrylamid, Methacrylamid, Acrylsäure-C₁-C₄-Alkylester und Methacrylsäure-C₁-C₄-Alkylester eingesetzt werden. Nichtionische, vollsynthetische Polymere, wie beispielsweise Polyvinylalkohol oder Polyvinylpyrrolidinon, sind ebenfalls als erfindungsgemäße Verdickungsmittel einsetzbar. Weiterhin bevorzugt werden natürlich vorkommende, gegebenenfalls modifizierte Verdickungsmittel eingesetzt. Dazu zählen z.B. Guargums, Skleroglucangums oder Xanthangums, pflanzliche Gums, wie Gummi arabicum, Ghatti-Gummi, Karaya-Gummi, Tragant-Gummi, Carrageen-Gummi, Agar-Agar, Johannisbrotkernmehl, Pektine, Alginate, Stärke, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen. Als anorganische Verdickungsmittel haben sich Schichtsilikate (polymere, kristalline Natriumdisilicate) als besonders geeignet im Sinne der vorliegenden Erfindung erwiesen. Insbesondere Tone, insbesondere Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate sind bevorzugt.

Es hat sich zur weiteren Verringerung des Irritationspotentials der erfindungsgemäßen Mittel als vorteilhaft herausgestellt, wenn die Mittel frei sind von organischen UV-Lichtschutzfiltersubstanzen. Unter UV-Lichtschutzfiltern sind organische Substanzen zu verstehen, die in der Lage sind, ultraviolette Strahlen zu absorbieren und die aufgenommene Energie in Form längerwelliger Strahlung, z. B. Wärme wieder abzugeben. Bevorzugt enthält das erfindungsgemäße Mittel keine UV-Filtersubstanzen aus den folgenden Substanzklassen: 3-Benzylidencampher und dessen Derivate, z. B. 3-(4-Methylbenzyliden)campher; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3-phenyl-zimtsäure-2-ethylhexylester (Octocrylene); Ester der Salicylsäure, vorzugsweise

Salicylsäure-2-ethylhexylester, Salicylsäure-4-isopropylben-zylester,

Salicylsäurehomomenthylester; Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxy-benzophenon; Ester der Benzalmalonsäure, vorzugsweise 4-Methoxybenzmalonsäuredi-2-ethylhexylester; Triazinderivate, wie z.B. 2,4,6-Trianilino-(p-carbo-2'-ethyl-1'-hexyloxy)-1,3,5-triazin und Octyltriazon; Propan-1,3-dione, wie z.B. 1-(4-*tert*-Butylphenyl)-3-(4'-methoxyphenyl)propan-1,3-dion oder 1-Phenyl-3-(4'-isopropylphenyl)-propan-1,3-dion; 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze. In einer weiteren Ausführungsform der vorliegenden Erfindung sind die erfindungsgemäßen Mittel frei von UV-Lichtschutzfiltern, ausgewählt aus der Gruppe, die gebildet wird aus 3-Benzylidencampher und dessen Derivaten, 4-Aminobenzoesäurederivaten, Zimtsäureestern, Salicylsäureestern, Benzophenonderivaten, Benzalmalonsäuren, Triazinderivaten, Propan-1,3-dionen, 2-Phenylbenzimidazol-5-sulfonsäuren, Benzophenonsulfonsäuren und Benzoylmethanderivaten. Frei von UV-Lichtschutzfiltern im Sinne der vorliegenden Erfindung bedeutet, dass die anwendungsbereiten Mittel weniger als 0,05 Gew.-%, bevorzugt weniger als 0,005 Gew.-% und ganz besonders bevorzugt weniger als 0,0001 Gew.-% der UV-Lichtschutzfilter, ausgewählt aus der Gruppe, die gebildet wird aus 3-Benzylidencampher und dessen Derivaten, 4-Aminobenzoesäurederivaten, Zimtsäureestern, Salicylsäureestern, Benzophenonderivaten, Benzalmalonsäuren, Triazinderivaten, Propan-1,3-dionen, 2-Phenylbenz-imidazol-5-sulfonsäuren, Benzophenonsulfonsäuren und Benzoylmethanderivaten, enthalten. Weiterhin hat es sich im Hinblick auf Reduktion der Hautirritation als vorteilhaft herausgestellt, wenn das erfindungsgemäße Mittel frei von Konservierungsmitteln auf Paraben-Basis ist. Parabene sind dem Fachmann als Konservierungsmittel für Lebensmittel wie auch für kosmetische Produkte bekannt. Unter Konservierungsmitteln auf Paraben-Basis sind im Sinne der vorliegenden Anmeldung die verzweigten und unverzweigten C₁-C₁₀-Alkylester der 4-Hydroxybenzoesäure sowie deren physiologisch verträglichen Salze, die sogenannten 4-Hydroxybenzoate, zu verstehen. Insbesondere bevorzugt sind die erfindungsgemäßen Mittel frei von Methyl-4-hydroxybenzoat (Methyl Paraben), Ethyl-4-hydroxybenzoat (Ethyl Paraben), Propyl-4-hydroxybenzoat (Propyl Paraben), 2-Propyl-4-hydroxybenzoat (Isopropyl Paraben), Butyl-4-hydroxybenzoat (Butyl Paraben), 2-Butyl-4-hydroxybenzoat (Isobutyl Paraben) und n-Heptyl-4-hydroxybenzoat (Heptyl Paraben) sowie den Natrium- und/oder Kaliumsalzen von Methyl-4-hydroxybenzoat. In einer bevorzugten Ausführungsform sind die erfindungsgemäßen Mittel daher frei von Konservierungsmitteln auf Paraben-Basis. Frei von Konservierungsmitteln auf Paraben-Basis im Sinne der vorliegenden Erfindung bedeutet, dass die anwendungsbereiten Mittel weniger als 0,05 Gew.-%, bevorzugt weniger als 0,005 Gew.-% und ganz besonders bevorzugt weniger als 0,0001 Gew.-% der Parabene, ausgewählt aus der Gruppe, die gebildet wird aus den verzweigten und unverzweigten C₁-C₁₀-Alkylestern der 4-Hydroxybenzoesäure sowie deren physiologisch verträglichen Salzen, enthält.

Schließlich hat es sich als vorteilhaft herausgestellt, wenn das erfindungsgemäße Mittel frei von Silikonen ist. Unter Silikonen sind im Sinne der vorliegenden Anmeldung flüchtige und nichtflüchtige Silikone zu verstehen. Insbesondere sind die erfindungsgemäßen Mittel frei von Silikonen, die ausgewählt sind aus
(i) Polyalkylsiloxanen (wie Dimethicone), Polyarylsiloxanen, Polyalkylarylsiloxanen, die jeweils flüchtig oder nicht flüchtig, geradkettig, verzweigt oder cyclisch (INCI-Bezeichnung Cyclomethicone), vernetzt oder nicht vernetzt sind;
(ii) Polysiloxanen, die in ihrer allgemeinen Struktur eine oder mehrere organofunktionelle Gruppen enthalten, die ausgewählt sind unter:
   substituierten oder unsubstituierten aminierten Gruppen (INCI-Bezeichnung:
      Amodimethicone u/o Trimethylsilylamodimethicone); (per)fluorierten Gruppen;
      Thiolgruppen; Carboxylatgruppen; hydroxylierten Gruppen (insb. Dimethiconcopolyole); alkoxylierten Gruppen; Acyloxyalkylgruppen; amphoteren Gruppen; Bisulfitgruppen;
      Hydroxyacylaminogruppen; Carboxygruppen; Sulfonsäuregruppen; und Sulfat- oder Thiosulfatgruppen;
(iii) linearen Polysiloxan(A)-Polyoxyalkylen(B)-Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Solche Silikone werden beispielsweise unter den Handelsnamen: Dow Corning 929 Emulsion (hydroxylamino-modifiziertes Silikon, Amodimethicone), DC 2-2078 (Hersteller Dow Corning, INCI-Bezeichung: Aminopropyl Phenyl Trimethicone), DC 5-7113 (Hersteller Dow Corning, INCI-Bezeichnung: Silicone Quaternium 16), SM-2059 (Hersteller: General Electric), SLM-55067 (Hersteller: Wacker), Abil-Quat 3270 und 3272 (Hersteller: Th. Goldschmidt; diquaternäre Polydimethylsiloxane, Quaternium-80), Q2-7224 (Hersteller: Dow Corning; ein stabilisiertes Trimethylsilylamodimethicon), DC 8500 (Bis(C13-15 Alkoxy) PG Amodimethicone, Fa. Dow Corning), Silcare Silicone SEA (Trideceth-9 PG-Amodimethicone, Fa. Clariant), SILWET (Union Carbide Corporation), Dow Corning 190 (Dimethiconpolyol) und Dow Corning 193 vertrieben. Frei von Silikonen im Sinne der vorliegenden Erfindung bedeutet dabei, dass die anwendungsbereiten Mittel weniger als 0,01 Gew.-%, bevorzugt weniger als 0,005 Gew.-% und ganz besonders bevorzugt weniger als 0,0001 Gew.-% an Silikonen, jeweils bezogen auf das Gesamtgewicht des anwendungsbereiten Mittels, enthalten.

Erfindungsgemäß bevorzugte Mittel sind frei von den oben genannten UV-Lichtschutzfiltern, frei von Konservierungsmitteln auf Paraben-Basis und/oder frei von Silikonen.

Ferner können die erfindungsgemäßen Mittel weitere Wirk-, Hilfs- und Zusatzstoffe enthalten, wie beispielsweise nichtionische Polymere, kationische Polymere, zwitterionische und amphotere Polymere, anionische Polymere, Strukturanten wie Glucose, Maleinsäure und Milchsäure, haarkonditionierende Verbindungen wie Phospholipide, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, bevorzugt Dimethicon, Farbstoffe zum Anfärben des Mittels, Antischuppenwirkstoffe, Wirkstoffe wie Aminosäuren, Oligopeptide und Proteinhydrolysate, Polyphenole und (Pseudo)Ceramide, Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, B₃, B₅, B₆, C, E, F und H, Pflanzenextrakte, Fette und Wachse wie Bienenwachs, Montanwachs und Paraffine, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel wie Ethylenglykolmono- und - distearat sowie PEG-3-distearat, Pigmente, Treibmittel wie Propan-Butan-Gemische, N₂O, Dimethylether, CO₂ und Luft und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Erfindungsgemäß bevorzugte Mittel zur Farb- und Formveränderung der Haare sind dadurch gekennzeichnet, dass sie einen möglichst neutralen pH-Wert besitzen. Eine weitere bevorzugte Ausführungsform der vorliegenden Erfindung besteht darin, dass das anwendungsbereite Mittel einen pH-Wert zwischen 4,0 und 12,0, bevorzugt zwischen 5,0 und 11,5, insbesondere bevorzugt zwischen 6,0 und 11,0 besitzt. Bei den pH-Werten im Sinne der vorliegenden Erfindung handelt es sich um pH-Werte, die bei einer Temperatur von 22 °C gemessen wurden. Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß bevorzugte Alkalisierungsmittel sind ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat, Kaliumcarbonat, 2-Aminoethan-1-ol (Monoethanolamin), Triethanolamin, Ammoniak, 1-Aminopropan-2-ol und 2-Amino-2-methylpropan-1-ol.

Die Konfektionierung der erfindungsgemäßen Farb- und Formveränderungsmittel unterliegt prinzipiell keinerlei Beschränkungen. Üblicherweise werden die erfindungsgemäßen Mittel als 1-Komponentenmittel konfektioniert, die gegebenenfalls unmittelbar vor der Anwendung mit einer zweiten Zubereitung, enthaltend beispielsweise ein Oxidationsmittel, vermischt werden. Es hat sich aber in einigen Fällen auch als bevorzugt erwiesen, wenn das Produkt als 2-Komponentenmittel konfektioniert ist. Im Rahmen einer bevorzugten Ausführungsform werden die erfindungsgemäßen Mittel daher derart konfektioniert, dass eine der erfindungswesentlichen Komponenten separat verpackt ist. Dabei spielt es erfindungsgemäß zunächst keine Rolle, welche der erfindungsgemäßen Komponenten separat verpackt wird; es kann aber bevorzugt sein, die Zubereitung, die das *trans*-4-*tert*.-Butyl-Cyclohexanol enthält, bis zur Anwendung separat zu verpacken. Erfindungsgemäß bevorzugte Mittel sind dadurch gekennzeichnet, dass das Mittel unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden und wobei ein Container ein Färbemittel enthält, welches in einem kosmetischen Träger mindestens ein Oxidationsfarbstoffvorprodukt und *trans*-4-*tert*.-Butyl-Cyclohexanol enthält, und ein weiterer Container eine Oxidationsmittelzubereitung, enthaltend mindestens ein Oxidationsmittel, enthält. Das Färbemittel besitzt dabei bevorzugt einen pH-Wert zwischen 5,0 und 12,0, bevorzugt zwischen 6,0 und 11,0.

Ein weiterer Gegenstand der vorliegenden Erfindung ist ein Verfahren zur Farb- und/oder Formveränderung keratinischer Fasern, bei dem ein erfindungsgemäßes Mittel gemäß obiger Vorgaben auf das Haar aufgetragen wird, für eine Einwirkzeit von 2 bis 60 Minuten, bevorzugt von 5 bis 45 Minuten auf dem Haar belassen wird, und anschließend das Haar ausgespült wird.

Eine bevorzugte Ausführungsform des erfindungsgemäßen Verfahrens zur Farbveränderung keratinischer Fasern liegt vor, wenn eine Zusammensetzung in einem kosmetischen Träger, enthaltend mindestens ein Oxidationsfarbstoffvorprodukt und *trans*-4-*tert*.-Butyl-Cyclohexanol mit einer Oxidationsmittelzubereitung, enthaltend Wasserstoffperoxid, zu einer homogenen Zusammensetzung vermischt, diese auf das Haar aufgebracht wird, für eine Einwirkzeit von 2 bis 60 Minuten, bevorzugt von 5 bis 45 Minuten auf dem Haar belassen wird, und anschließend das Haar ausgespült wird. Die Anwendungstemperaturen bei der erfindungsgemäßen Farbveränderung keratinischer Fasern können in einem Bereich zwischen 15 und 45 °C liegen. Nach einer Einwirkungszeit wird das Haarfärbemittel durch Ausspülen von dem zu färbenden Haar entfernt. Das Nachwaschen mit einem Shampoo kann entfallen, wenn ein stark tensidhaltiger Träger, z. B. ein Färbeshampoo, verwendet wurde.

Eine weitere bevorzugte Ausführungsform des erfindungsgemäßen Verfahrens zur dauerhaften Formveränderung keratinischer Fasern liegt vor, wenn eine Zusammensetzung in einem kosmetischen Träger, enthaltend mindestens eine keratinreduzierende Substanz sowie *trans*-4-*tert.-*Butyl-Cyclohexanol auf das Haar aufgebracht wird, für eine Einwirkzeit von 2 bis 45 Minuten, bevorzugt von 10 bis 30 Minuten auf dem Haar belassen wird, und anschließend gegebenenfalls das Haar ausgespült wird. Anschließend wird ein Fixiermittel, enthaltend mindestens ein Oxidationsmittel, vorzugsweise Wasserstoffperoxid, sowie vorzugsweise zusätzlich mindestens einen Stabilisator oder Komplexbildner, auf das Haar aufgebracht, für eine Einwirkzeit von 2 bis 45 Minuten, bevorzugt von 10 bis 30 Minuten auf dem Haar belassen, und anschließend das Haar ausgespült wird. Die Anwendungstemperaturen bei der erfindungsgemäßen dauerhaften Formveränderung keratinischer Fasern können in einem Bereich zwischen 15 und 45 °C liegen. Nach einer Einwirkungszeit wird das Fixiermittel durch Ausspülen von dem Haar entfernt. Das Nachwaschen mit einem Shampoo kann entfallen, wenn ein stark tensidhaltiger Träger verwendet wurde.

Bezüglich weiterer bevorzugter Ausführungsformen der erfindungsgemäßen Verfahren gilt mutatis mutandis das zu den erfindungsgemäßen Mitteln Gesagte.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Das anwendungsbereite Mittel wird bei solchen Systemen vom Anwender oder Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein weiterer Gegenstand der vorliegenden Erfindung ist daher eine Mehrkomponentenverpackungseinheit (Kit-of-Parts), enthaltend mindestens zwei getrennt voneinander konfektionierte Container, wobei ein Container mindestens eine farb- und/oder formverändernde Verbindung und *trans*-4-*tert*.-Butyl-Cyclohexanol enthält und ein Container eine Oxidationsmittelzusammensetzung, enthaltend mindestens ein chemisches Oxidationsmittel, insbesondere Wasserstoffperoxid, enthält. Eine bevorzugte Ausführungsform dieses Gegenstand der vorliegenden Erfindung ist eine Mehrkomponentenverpackungseinheit (Kit-of-Parts), enthaltend mindestens zwei getrennt voneinander konfektionierte Container, wobei ein Container eine Färbemischung in einem kosmetischen Träger, enthaltend mindestens eine farbgebende Komponente, insbesondere mindestens ein Oxidationsfarbstoffvorprodukt, *trans*-4-*tert*.-Butyl-Cyclohexanol, und ein Container eine Oxidationsmittelzusammensetzung, enthaltend mindestens ein chemisches Oxidationsmittel, insbesondere Wasserstoffperoxid, enthält.

Wird eine besonders starke Aufhellwirkung durch Einsatz von Persulfatsalzen bzw. Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen.

Eine weitere bevorzugte Ausführungsform des Erfindungsgegenstands ist eine Mehrkomponentenverpackungseinheit (Kit-of-Parts), enthaltend mindestens zwei getrennt voneinander konfektionierte Container, wobei ein Container eine Formveränderungsmischung in einem kosmetischen Träger, enthaltend mindestens eine formverändernde Komponente und *trans*-4-*tert.-*Butyl-Cyclohexanol, und ein Container eine Oxidationsmittelzusammensetzung, enthaltend mindestens ein chemisches Oxidationsmittel, insbesondere Wasserstoffperoxid, enthält. Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Anmischhilfe, wie beispielsweise eine Schale, eine Applikationshilfe, wie beispielsweise ein Kamm oder ein Pinsel, und/oder eine persönliche Schutzausrüstung, wie beispielsweise Einweg-Handschuhe dem Kit beigefügt ist. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der Erfindung ist die Verwendung der Mittel des ersten Erfindungsgegenstandes zur Verringerung der Schädigung der Haarstruktur bei der Farb- und/oder dauerhaften Formveränderung menschlicher Haare.

Ein weiterer Gegenstand der Erfindung ist schließlich die Verwendung der Mittel des ersten Erfindungsgegenstandes zur Verbesserung der Hautverträglichkeit bei der Farb- und/oder dauerhaften Formveränderung menschlicher Haare.

Ein weiterer Gegenstand der Erfindung ist schließlich die Verwendung eines erfindungsgemäßen Mittels zur Verringerung der Schädigung der Haarstruktur bei der Farb- und/oder dauerhaften Formveränderung menschlicher Haare.

Bezüglich weiterer bevorzugter Ausführungsformen der erfindungsgemäßen Verwendungen gilt mutatis mutandis das zu den erfindungsgemäßen Mitteln Gesagte.

Die nachfolgenden Beispiele sollen die vorliegende Erfindung verdeutlichen, ohne sie auf darauf einzuschränken.

### Beispiele

### 1) Färbecremes

| Rohstoff | E1 | E2 | E3 | E4 | E5 | E6 | E7 | E8 |
|---|---|---|---|---|---|---|---|---|
| Lanette D | 6,60 | 6,60 | 6,60 | 6,60 | 6,60 | 5,50 | -- | -- |
| Lorol techn. | 2,40 | 2,40 | 2,40 | 2,40 | 2,43 | 2,00 | -- | -- |
| Ammonium Carbomer, 1% | -- | -- | -- | -- | -- | -- | 12,00 | 12,00 |
| Lanette E, Pulver | -- | -- | -- | -- | -- | -- | 0,56 | 0,56 |
| Texapon NSF, 27% | -- | -- | -- | -- | -- | -- | 3,52 | 3,52 |
| Kalium Oleat, 12,5% | -- | -- | -- | -- | -- | -- | 2,40 | 2,40 |
| Cutina GMS SE | -- | -- | -- | -- | -- | -- | 1,60 | 1,60 |
| Cutina AGS | -- | -- | -- | -- | -- | -- | 1,60 | 1,60 |
| Eutanol G | -- | -- | -- | -- | -- | -- | 1,60 | 1,60 |
| Hydrenol D | -- | -- | -- | -- | -- | -- | 9,60 | 9,60 |
| Phospholipid EFA | -- | -- | -- | -- | -- | -- | 0,10 | 0,10 |
| Eumulgin B 2 | 0,60 | 0,60 | 0,60 | 0,60 | 0,60 | 0,50 | 2,40 | 2,40 |
| Eumulgin B 1 | 0,60 | 0,60 | 0,60 | 0,60 | 0,60 | 0,50 | -- | -- |
| Lamesoft PO 65 | 2,00 | 2,00 | 2,00 | 2,00 | 2,00 | 2,00 | -- | -- |
| Akypo Soft 45HP | 10,0 0 | 10,00 | 10,00 | 10,00 | 10,00 | 10,00 | -- | -- |
| Texapon K 14 S Special, 70 % | 2,80 | 2,80 | 2,80 | 2,80 | 2,80 | 2,80 | 1,38 | 1,38 |
| Produkt W 37194 | 3,75 | 3,75 | 3,75 | 3,75 | 3,75 | 3,75 | -- | -- |
| p-Toluylendiaminsulfat | 0,300 | 0,822 | 0,029 | -- | 1,533 | 1,161 | -- | -- |
| Resorcin | 0,350 | 0,416 | 0,011 | -- | 0,410 | 0,298 | 0,53 | 0,53 |
| 2-Methylresorcin | 0,24 6 | -- | -- | 0,004 | 0,343 | 0,128 | -- | -- |
| 5-Amino-2-methylphenol | -- | 0,023 | 0,001 | -- | -- | -- | -- | -- |
| 2-Amino-4-hydroxyethylamino-anisolsulfat | -- | -- | -- | -- | -- | -- | 0,02 | 0,02 |
| 4-Amino-3-methylphenol | 0,460 | 0,019 | -- | -- | -- | -- | -- | -- |
| 2-Chlor-6-methyl-3-aminophenol | -- | 0,047 | -- | -- | -- | -- | -- | -- |
| Bis-(5-Amino-2-hydroxyphenyl)methan 2HCl | -- | 0,539 | -- | 0,010 | -- | -- | -- | -- |
| 2,4-Diaminophenoxyethanol 2HCl | -- | -- | 0,002 | -- | -- | -- | -- | -- |
| 3-Aminophenol | -- | -- | 0,002 | -- | -- | 0,058 | 0,16 | 0,16 |
| 2,7-Dihydroxynaphthalin | -- | -- | -- | 0,104 | -- | -- | -- | -- |
| 2,4,5,6-Tetraaminopyrimidinsulfat | -- | -- | -- | 0,146 | -- | -- | -- | -- |
| 2-Amino-3-hydroxypyridin | -- | -- | -- | -- | 0,046 | 0,078 | -- | -- |
| Aerosil 200 | -- | -- | -- | -- | -- | -- | 0,25 | 0,25 |
| Ammoniumsulfat, techn. rein | 0,82 | 0,51 | 1,00 | 0,92 | -- | 0,50 | -- | -- |
| Natriumsulfit, wasserfrei, 96% | 0,40 | 0,40 | 0,40 | 0,40 | 0,40 | 0,40 | 0,20 | 0,20 |
| HEDP, 60% | 0,20 | 0,20 | 0,20 | 0,20 | -- | 0,20 | -- | -- |
| Na₄-EDTA, Pulver, 87% | -- | -- | -- | -- | -- | -- | 0,20 | 0,20 |
| Natriumhydroxid, 45% | 1,00 | 1,12 | 0,10 | 0,30 | 1,70 | -- | -- | -- |
| Kaliumhydroxid, 50% | -- | -- | -- | -- | -- | 0,90 | 0,85 | 0,85 |
| Ascorbinsäure | -- | -- | -- | -- | -- | 0,05 | 0,05 | 0,05 |
| Natriumsilikat 40/42 | 0,50 | 0,50 | 0,50 | 0,50 | 0,50 | 0,50 | -- | -- |
| Hydrovance | -- | -- | -- | -- | -- | -- | 2,00 | 2,00 |
| L-Serin | 1,00 | 1,00 | 1,00 | 1,00 | 1,00 | -- | 0,50 | 0,50 |
| *trans*-4-*tert* Butyl-Cyclohexanol | 0,40 | 0,55 | 0,65 | 0,75 | 2,00 | 1,10 | 2,50 | 0,75 |
| Ronacare Ectoin | -- | 0,50 | 1,00 | -- | 0,50 | 1,00 | -- | 0,50 |
| Ammoniak, 25 % | 7,00 | 6,50 | 7,00 | 7,00 | 6,50 | 7,50 | 6,00 | 6,00 |
| Parfum | qs | qs | qs | qs | qs | qs | qs | qs |
| Wasser, entsalzt | add 100 | | | | | | | |

| | | | | | | | | |
|---|---|---|---|---|---|---|---|---|
| Lanette D C₁₆-C₁₈-Fettalkohol (INCl-Bezeichnung: Cetearyl alcohol) (Cognis) Lorol tech. C₁₂-C₁₈-Fettalkohol (INCl-Bezeichnung: Coconut alcohol) (Cognis) Eumulgin B2 C₁₆-C₁₈-Fettalkohol, ethoxyliert (20 EO) (INCI-Bezeichnung: Ceteareth-20) (Cognis) Eumulgin B1 C₁₆-C₁₈-Fettalkohol, ethoxyliert (12 EO) (INCI-Bezeichnung: Ceteareth-12) (Cognis) Lamesoft PO 65 C₁₂-C₁₈-Alkylpolyglucosid, Glycerylmonooleat (ca. 66 %, INCI-Bezeichnung: Coco-Glucoside, Glyceryl Oleate, Aqua) (Cognis) Akypo Soft 45HP C₁₂-C₁₄-Alkylether, ethoxyliert (6 EO) Carbonsäure, Natriumsalz (ca. 21 %, INCI-Bezeichnung: Sodium Laureth-6 Carboxylate, Aqua) (KAO) Texapon K14 S Special C₁₂-C₁₄-Alkylethersulfat, ethoxyliert (3 EO), Natriumsalz (ca. 70 %, INCI-Bezeichnung: Sodium Myreth Sulfate, Aqua) (Cognis) Produkt W 37194 Copolymer aus Acrylsäure, Natriumsalz und Trimethylammoniopropylacrylamidchlorid (ca. 20 %, INCI-Bezeichnung: Acrylamidopropyltrimonium Chloride/Acrylates Copolymer, Aqua) (Stockhausen) Natriumsilikat 40/42 Natronwasserglas Ronacare Ectoin (4S)-1,4,5,6-Tetrahydro-2-methyl-pyrimidin-4-carbonsäure (INCI-Bezeichnung: Ectoin) (Rona) Lanette E, Pulver C₁₆-C₁₈-Fettalkoholsulfat, Natrium-Salz (INCI-Bezeichnung: Sodium Cetearyl Sulfate) (Cognis) Texapon NSF, 27% C₁₂-Fettalkoholsulfat, ethoxyliert (2 EO) Natrium-Salz (INCI-Bezeichnung: Sodium Laureth Sulfate) (Cognis) Cutina GMS SE Stearinsäureglycerylester (INCI-Bezeichnung: Glyceryl Stearate) (Cognis) Cutina AGS Ethylenglycoldistearylester (INCI-Bezeichnung: Glycol Distearate) (Cognis) Eutanol G 2-Octyldodecanol (INCI-Bezeichnung: Octyldodecanol) (Cognis) Hydrenol D C₁₆-C₁₈-Fettalkohol (INCI-Bezeichnung: Cetearyl alcohol) (Cognis) Phospholipid EFA zwitterionisches Phospholid (INCI-Bezeichnung: Linoleamidopropyl PGdimonium chloride phosphate) (Uniqema) Aerosil 200 Siliciumdioxid, pyrogen (INCI-Bezeichnung: Silica) (Evonik) Hydrovance Hydroxyethylharnstoff (ca. 50 %; INCI-Bezeichnung: Hydroxyethylura, Aqua) (National Starch) | | | | | | | | |

| Rohstoff | E9 | E10 | E11 | E12 |
|---|---|---|---|---|
| Cetiol V | 2,30 | 2,30 | 2,30 | 2,30 |
| Xanthan FN | 0,05 | 0,05 | 0,05 | 0,05 |
| Lanette N | 14,00 | 14,00 | 14,00 | 14,00 |
| Lanette D | 3,90 | 3,90 | 3,90 | 3,90 |
| Cutina GMS SE | 6,00 | 6,00 | 6,00 | 6,00 |
| Kokosamidopropylbetain, 40 % | 2,00 | 2,00 | 2,00 | 2,00 |
| Monoethanolamin | 0,30 | 0,30 | 0,30 | 0,30 |
| Natriumsulfit wasserfrei, 96% | 0,20 | 0,20 | 0,20 | 0,20 |
| RonaCare Ectoin | 1,00 | 1,00 | -- | -- |
| *trans*-4-*tert* Butyl-Cyclohexanol | 0,55 | 2,50 | 0,80 | 1,00 |
| Ascorbinsäure | 0,05 | 0,05 | 0,05 | 0,05 |
| Ammoniak, 25 % | 6,00 | 6,00 | 6,00 | 6,00 |
| 4,5-Diamino-1-(2-hydroxyethyl)pyrazolsulfat | 1,00 | 1,00 | 1,00 | 1,00 |
| 5-Amino-2-methylphenol | 0,20 | 0,20 | 0,20 | 0,20 |
| 2-Amino-3-hydroxypyridin | 0,20 | 0,20 | 0,20 | 0,20 |
| 2-Amino-6-chloro-4-nitrophenol | 0,30 | -- | 0,30 | -- |
| Wasser, vollentsalzt | add 100 | | | |

| | | | | |
|---|---|---|---|---|
| Cetiol V Ölsäuredecylester (INCI-Bezeichnung: Decyl Oleate) (Cognis) Xanthan FN Xanthan Gum (INCI-Bezeichnung: Xanthan Gum) (Jungbunzlauer) Lanette N C₁₆-C₁₈-Fettalkohol, Natrium C₁₆-C₁₈-Fettalkoholsulfat (INCI-Bezeichnung: Cetearyl alcohol, Sodium Cetearyl Sulfate) (Cognis) | | | | |

Die Fettbasis wurde jeweils zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.

### 2) Entwicklerzubereitungen

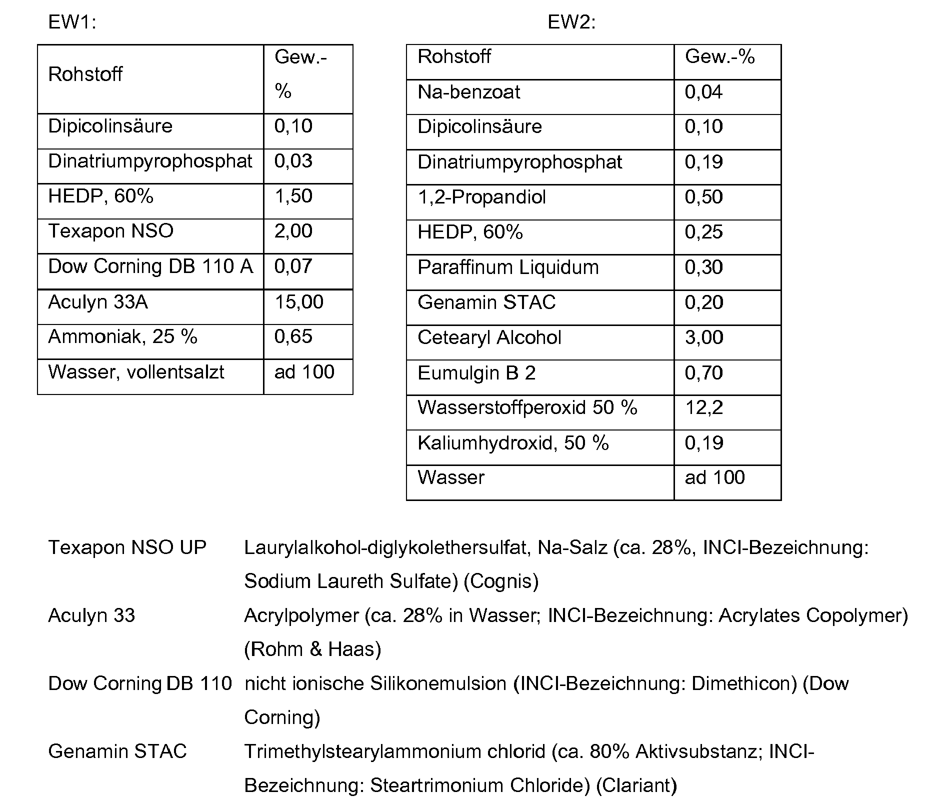

### 3) Ausfärbungen:

Die Färbecremes E1 bis E6 wurden vor der Anwendung mit einer Entwicklerlösung EW1 im Gewichtsverhältnis von 1:1 versetzt und innig vermischt. Die Färbecremes E7 und E8 wurden vor der Anwendung mit einer Entwicklerlösung EW2 im Gewichtsverhältnis von 1:1 versetzt und innig vermischt. Die Färbecremes E9 bis E12 wurden vor der Anwendung mit einer 6 Gew.-%igen Wasserstoffperoxidzubereitung im Gewichtsverhältnis von 1:2 versetzt und innig vermischt. Pro Gramm Haar (europäisches Humanhaar, Alkinco 6634, #10/2003, A9) wurden 4 g des frisch hergestellten, anwendungsbereiten Färbemittels aufgetragen. Die Einwirkzeit betrug 30 min bei 35°C für die Färbemittel. Danach wurden die Strähnen 30 s lang mit warmem Wasser ausgespült und luftgetrocknet. Die gefärbten Strähnen zeichneten sich durch glänzende Farben und einen angenehmen Griff aus.

## Patentansprüche

1. Mittel zur Farb- und/oder Formveränderung von keratinischen Fasern, insbesondere menschlichen Haaren, enthaltend in einem kosmetischen Träger mindestens eine farb- und/oder formverändernde Komponente, **dadurch gekennzeichnet, dass** das Mittel zusätzlich *trans*-4-*tert*-Butyl-Cyclohexanol enthält.

2. Mittel nach Anspruch 1, **dadurch gekennzeichnet, daß** es - bezogen auf sein Gewicht-0,05 bis 10 Gew.-%, vorzugsweise 0,1 bis 7,5 Gew.-%, weiter bevorzugt 0,25 bis 5 Gew.-%, besonders bevorzugt 0,5 bis 2,5 und insbesondere 0,5 bis 1,25 Gew.-% *trans*-4-*tert*-Butyl-Cyclohexanol enthält.

3. Mittel nach einem der Ansprüche 1 bis 2, **dadurch gekennzeichnet, daß** das Mittel als farbverändernde Komponente mindestens ein Oxidationsfarbstoffvorprodukt und/oder mindestens einen direktziehenden Farbstoff und/oder mindestens einer Vorstufe naturanaloger Farbstoffe und/oder mindestens ein Aufhellmittel enthält.

4. Mittel zum Färben und/oder Aufhellen keratinischer Fasern nach einem der Ansprüche 1 bis 3, **dadurch gekennzeichnet, daß** es 0,5 bis 15 Gew.-%, vorzugsweise 1 bis 12,5 Gew.-%, besonders bevorzugt 2,5 bis 10 Gew.-% und insbesondere 3 bis 7 Gew.-% Wasserstoffperoxid (berechnet als 100%iges H₂O₂) enthält.

5. Mittel nach einem der Ansprüche 1 bis 4, **dadurch gekennzeichnet, daß** es als farbverändernde Komponente mindestens ein Oxidationsfarbstoffvorprodukt enthält, wobei der Gewichtsanteil aller Oxidationsfarbstoffvorprodukte zwischen 0,001 und 20 Gew.-%, bevorzugt zwischen 0,1 und 10 Gew.-% und insbesondere zwischen 0,2 und 5,0 Gew.-%, jeweils bezogen auf das Gesamtgewicht des anwendungsbereiten Mittels, liegt.

6. Mittel zum Färben und/oder Aufhellen keratinischer Fasern nach Anspruch 5, **dadurch gekennzeichnet, daß** es als Oxidationsfarbstoffvorprodukt mindestens eine Entwicklerkomponente enthält, wobei bevorzugte Entwicklerkomponenten ausgewählt sind aus p-Phenylendiamin, p-Toluylendiamin, , N,N-Bis-(2-hydroxyethyl)amino-p-phenylendiamin, 1,3-Bis-[(2-hydroxyethyl-4'-aminophenyl)amino]-propan-2-ol, Hydroxyethyl-p-Phenylendiamin, 1,10-Bis-(2',5'- diaminophenyl)-1,4,7,10-tetraoxadecan, 4-Aminophenol, 4-Amino-3-methylphenol, Bis-(5- amino-2-hydroxyphenyl)methan, 2,4,5,6- Tetraaminopyrimidin, 2-Hydroxy-4,5,6- triaminopyrimidin, 4,5-Diamino-1-(2-hydroxyethyl)-pyrazol.

7. Mittel zum Färben und/oder Aufhellen keratinischer Fasern nach einem der Ansprüche 5 oder 6, **dadurch gekennzeichnet, daß** es als Oxidationsfarbstoffvorprodukt mindestens eine Kupplerkomponente enthält, wobei bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

8. Mittel zum Färben und/oder Aufhellen keratinischer Fasern nach einem der Ansprüche 5 bis 7, **dadurch gekennzeichnet, daß** es mindestens einen direktziehenden Farbstoff enthält, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol

9. Verwendung eines Mittels gemäß einem der Ansprüche 1 bis 8 zur Verbesserung der Hautverträglichkeit bei der Farb- und/oder dauerhaften Formveränderung menschlicher Haare.

10. Verwendung eines Mittels gemäß einem der Ansprüche 1 bis 8 zur Verringerung der Schädigung der Haarstruktur bei der Farb- und/oder dauerhaften Formveränderung menschlicher Haare.
